# EUROPÄISCHE PATENTSCHRIFT

(11) **EP 1 616 555 B1**
(45) Veröffentlichungstag und Bekanntmachung des Hinweises auf die Patenterteilung: **10.03.2010**
(21) Anmeldenummer: 05022016.9
(22) Anmeldetag: 09.05.2000
(51) Int. Cl.: A61K 8/11, A61K 8/60, A61K 8/19, A61K 8/81, A61K 8/73, A61Q 5/00, A61Q 5/02, A61Q 5/04, A61Q 19/10

(54) **Instantgel-Zubereitung und Verwendung von festen, gasifizierten Teilchen als akustischer Indikator**
Preparation of an instant gel and the use of gaseous solid particles as acoustic indicator
Préparation d'un gel instantané et utilisation de particules solides gazifiées comme indicator acoustique

(30) Priorität: 19.04.2000 DE 10019313; 09.06.1999 DE 19926316
(43) Veröffentlichungstag der Anmeldung: 18.01.2006
(62) Teilanmeldung aus: 00109775.7
(73) Patentinhaber: The Procter & Gamble Company, Cincinnati OH 45202 (US)
(72) Erfinder: Birkel, Susanne Dr., 64285 Darmstadt (DE); Wendel, Harald, 64372 Ober-Ramstadt (DE); Steinbrecht, Karin Dr., 64372 Ober-Ramstadt (DE); Maurer, Wolfgang, 69198 Schriesheim (DE)
(74) Vertreter: Hirsch, Uwe Thomas M.H.

(56) Entgegenhaltungen:
- DE-A1- 19 745 964
- FR-A- 2 753 097
- US-A- 2 971 292
- PATENT ABSTRACTS OF JAPAN Bd. 014, Nr. 334 (C-0742), 18. Juli 1990 (1990-07-18) & JP 02 121917 A (LION CORP), 9. Mai 1990 (1990-05-09)
- PATENT ABSTRACTS OF JAPAN Bd. 014, Nr. 197 (C-0712), 23. April 1990 (1990-04-23) & JP 02 040319 A (TADAO SHIRAISHI), 9. Februar 1990 (1990-02-09)

## Beschreibung

Gegenstand der vorliegenden Erfindung ist eine Instantgel-Zubereitung aus einem Gemisch von festem Gelbildner und einem festen, bei Kontakt mit Feuchtigkeit einen akustisch wahrnehmbaren Effekt produzierenden Stoff sowie die Verwendung von gasifizierten Teilchen als akustischer Indikator zur Anzeige der Gebrauchsfertigkeit einer Instantzubereitung nach Flüssigkeitszugabe. Die gasifizierten Teilchen enthalten mindestens ein in einer geeigneten Umhüllung eingeschlossenes Gas. Bei Kontakt der Umhüllung auf Saccharidbasis mit Feuchtigkeit oder Wasser wird das Gas freigesetzt. Die Freisetzung des Gases kann von einem besonderen sensorischen, insbesondere taktilen oder akustischen Effekt (Popp-Effekt) begleitet sein.

Die der Erfindung zugrunde liegende Aufgabe bestand darin, ein Verfahren gemäß Anspruch 10 zur Herstellung kosmetischer Mittel mit neuen, ausgefallenen Eigenschaften zur Verfügung zu stellen. Eine solche Eigenschaft ist beispielsweise ein bei Anwendung des kosmetischen Mittels auftretender sensorischer, insbesondere taktiler oder akustischer Effekt. Durch diese neuen, ausgefallenen Eigenschaften sollen die üblichen, kosmetischen Wirkungen allerdings nicht wesentlich beeinträchtigt, sondern idealerweise sogar noch verstärkt werden.

Instantzubereitungen zeichnen sich dadurch aus, dass sie im wesentlichen aus festen, meist pulver- oder granulatförmigen Stoffen bestehen, welche sich bei Zugabe von Flüssigkeit, insbesondere Wasser, unter Rühren leicht lösen und eine gebrauchsfertige Mischung ergeben. Kommt es bei der gebrauchsfertigen Mischung für eine optimale Anwendbarkeit auf die Konsistenz an, so ist es für den Anwender nicht immer leicht, den Zeitpunkt zu erkennen, wann die gewünschte Konsistenz erreicht ist. Die der Erfindung zugrunde liegende Aufgabe bestand darin, dem Anwender von Instantprodukten diesen Zeitpunkt anzuzeigen bzw. das Erkennen dieses Zeitpunktes zu erleichtern.

Auf dem Gebiet der Süßwaren sind die sogenannten 'popping candies' oder 'gasified candies' bekannt, welche bei Kontakt mit Feuchtigkeit oder Speichel durch Freisetzung eines unter Druck eingeschlossenen Gases einen spürbaren oder akustischen Effekt (popping) erzeugen. Diese popping candies werden in einer Vielzahl von Patentanmeldungen beschrieben. Stellvertretend seien hier die US 3,012,893, die US 4,262,029, die US 4,275,083, die EP 0 017 691, die EP 0 326 692, die EP 0 533 609 und die WO 86/01376 sowie die in diesen Dokumenten jeweils zitierte Literatur genannt. Eine Beschreibung des dem Popp-Effekt zugrundeliegenden Mechanismus sowie der Parameter, welche die Quantität, Qualität und Zeitverzögerung des Poppeffektes bestimmen, ist in der EP 0 533 609 enthalten. Bei der Herstellung von Popping Candy wird in der Regel Zucker geschmolzen und ein Gas, vorzugsweise Kohlendioxid, in der Schmelze dispergiert. Wenn die Schmelze abgekühlt wird, erstarrt der Zucker, das dispergierte Gas wird eingeschlossen und es bildet sich ein fester Schaum. Hieraus kann ein Granulat hergestellt werden, welches Gasblasen mit einer Umhüllung aus einer festen Zuckerschicht enthält und wobei der Gasdruck in der Blase größer ist als der Umgebungsdruck. Die Einarbeitung des Gases kann auch unter Überdruck in einem Autoklaven erfolgen. Bei Kontakt der Umhüllung mit Feuchtigkeit löst sich die feste Umhüllung soweit auf, bis die Stabilität der Umhüllung bzw. die Oberflächenspannung geringer ist als die durch den Innendruck hervorgerufene Kraft. Dann platzt die Umhüllung auf und das Gas entweicht unter Erzeugung eines akustisch oder taktil wahrnehmbaren Effektes.

Die FR-A-2753097 oder die US-A-2971292 offenbaren Instantgel-Zubereitungen bestehend aus einem Gemisch von Trockensubstanzen und Gas-produzierendem Stoff. In der DE-A-1974564 wird eine Instantshampoo-Zubereitung beschrieben, die pulverförmige Tenside und Gas-produzierenden Stoffe wie sauer reagierende Verbindungen und Carbonat oder Bicarbonat, enthält. Weiterhin werden in der JP-A-02121917 oder JP-A-02040319 kosmetische Mittel mit einem Gehalt an gasifizierten Teilchen erwähnt, die jedoch keine Instandgel-Zubereitungen sind.

Es wurde nun gefunden, dass gasifizierte Teilchen für die Herstellung von Instantgel-Zubereitungen eingesetzt werden können. Gegenstand der Erfindung ist daher eine Instantgel-Zubereitung bestehend aus einem Gemisch von Trockensubstanzen, wobei das Gemisch einen Gehalt aufweist an
(A) mindestens einem Gelbildner und oder mindestens einem superabsorbierenden Polymer und
(B) mindestens einem festen, bei Kontakt mit Feuchtigkeit einen akustisch wahrnehmbaren Effekt produzierenden Stoff, ausgewählt aus gasifizierten Teilchen, welche mindestens ein in einer festen Umhüllung eingeschlossenes Gas enthalten, wobei die Umhüllung so gewählt ist, dass das Gas bei Kontakt der Umhüllung mit Wasser oder Feuchtigkeit freigesetzt wird und das Material der Umhüllung ausgewählt ist aus Materialien auf Saccharidbasis, und wobei das in den gasifizierten Teilchen eingeschlossene Gas ausgewählt ist aus Kohlendioxid, Stickstoff, Luft oder aus Mischungen dieser Gase und wobei der Druck des in den Teilchen eingeschlossenen Gases größer ist als der Umgebungsdruck.

Als eingeschlossene Gase sind inerte Gase oder Gasgemische geeignet ausgewählt aus Kohlendioxid, Sauerstoff, Stickstoff oder Luft, von denen Kohlendioxid besonders bevorzugt ist, da es die intensiveren Geräuscheffekte liefert.

Das umhüllende Material ist auf Zuckerbasis, d.h. auf Basis von Mono-, Oligo- oder Polysacchariden. Insbesondere können Zucker wie Saccharose, Lactose, Glucose, Dextrose, Maltose, Fructose, Disaccharide, Trisaccharide, Tetrasaccharide, Pentasaccharide, Hexasaccharide und höhere Oligo- oder Polysaccharide oder Zuckerstoffe wie Sorbitol oder deren Mischungen verwendet werden. Bei Verwendung von Sorbitol wird aufgrund der langsameren Wasserlöslichkeit eine Verzögerung der Gasfreisetzung erreicht. Eine verzögerte Gasfreisetzung bzw. eine damit verbundene Verlängerung des Effektes kann aber auch erreicht werden, indem die gasifizierten Teilchen in Kombination mit einer wasserhaltigen, hochviskosen, insbesondere gelförmigen Zusammensetzung, mit der sie kurz vor der Anwendung vermischt oder in Kontakt gebracht werden, angewendet werden. Vorzugsweise wird eine Mischung von verschiedenen Zuckern eingesetzt, z.B. eine Mischung aus Saccharose, Lactose und Glucose. Der Gehalt an eingeschlossenem Gas beträgt vorzugsweise 0,05 bis 15, besonders bevorzugt 0,3 bis 2,0 cm³ pro Gramm Gesamtmasse. Die Teilchengröße beträgt vorzugsweise 0,1 bis 5 mm, besonders bevorzugt von 0,5 bis 4,5 mm. Die Teilchen können mit einem geeigneten Material beschichtet sein, z.B. mit Fetten, Schellack, Gelatine, oder Cellulosederivaten wie Hydroxyalkylcellulosen, insbesondere Hydroxymethyl-, Hydroxyethyl- oder Hydroxypropylcellulose. Das Beschichtungsmaterial ist vorzugsweise hydrophob. Die Herstellung von geeigneten Granulaten von in Zuckerumhüllungen eingeschlossenen Gasen ist z.B. beschrieben in US 3,012,893, US 4,262,029, EP 0 533 609, EP 0 017 691 sowie der dort jeweils zitierten Literatur. Geeignete Granulate sind auch im Handel erhältlich, beispielsweise Popping Candy der Firma Zeta Espacial S.A., Rubi/Spanien. Die Herstellung von beschichteten, gasifizierten Teilchen ist in der US 4,275,083 beschrieben.

Das aus den Instantgel-Zubereitungen herstellbare kosmetische Mittel besteht vorzugsweise aus zwei bis unmittelbar vor der Anwendung getrennt voneinander vorliegenden Teilen, wobei ein erster Teil mindestens einen kosmetischen Wirkstoff in einer wasserhaltigen, kosmetischen Basis enthält und ein übliches kosmetisches Präparat wie z.B. ein Shampoo, eine Haarkur, ein Haarstylinggel oder ein Haarstylingschaum sein kann. Der zweite Teil besteht aus oder enthält Teilchen, die mindestens ein Gas in einer festen Umhüllung auf Saccharidbasis enthalten. Die zwei Teile können entweder in einer Zweikomponentenverpackung, z.B. einer handelsüblichen Zweikammerverpackung oder in einem aus zwei separaten Verpackungen bestehenden Set verpackt sein. Die beiden separat verpackten Teile des Mittels werden unmittelbar vor der Anwendung miteinander vermischt oder zumindest miteinander in Kontakt gebracht.

Die gasifizierten Teilchen machen vorzugsweise 1 bis 75, besonders bevorzugt 3 bis 30 Gewichtsprozent des gesamten kosmetischen Mittels aus. Im Prinzip können die gasifizierten Teilchen in Kombination mit jedem beliebigen kosmetischen Mittel mit wasserhaltiger Formulierung als sensorischer, insbesondere akustischer und/oder taktiler Effektgeber eingesetzt werden. Das kosmetische Mittel kann in Form von Haar- oder Körperreinigungsmitteln, Haarpflegemitteln, Haarfestigungs-, Haarfärbe- oder Tönungsmitteln, Blondiermitteln sowie als Dauerwellmittel vorliegen. Das Mittel kann als Lotion, Schaum, Milch, Gel, Creme oder Gelschaum oder in Form eines emulsionsförmigen Haarpflegemittels (Haarspülung, Conditioner) appliziert werden.

Durch Kontakt mit Wasser oder Feuchtigkeit wird die Zuckerumhüllung gelöst und das eingeschlossene Gas wird freigesetzt. Dies ist beispielsweise der Fall, wenn wasserhaltige kosmetische Formulierungen wie Shampoos, Kuren, Stylinggele oder Stylingschäume oder auch die feuchten Haare selbst damit in Berührung kommen. Dadurch, dass die Zuckerumhüllung selbst eine hohe Affinität zum Haar hat, kann nach einer entsprechenden Haarbehandlung und nach dem Auswaschen beispielsweise des Shampoos oder der Kur noch ein positiver Volumeneffekt des behandelten Haars festgestellt werden.

Das aus den Instantgel-Zubereitungen herstellbare Mittel kann auch in Form eines im wesentlichen wasserfreien Einkomponentenmittels vorliegen. Liegt es beispielsweise in Form eines Trockenshampoos vor, so enthält es zusätzlich mindestens ein waschaktives Tensid oder ein Tensidgemisch.

Das aus den Instantgel-Zubereitungen herstellbare Mittel kann die für Haarbehandlungsmittel üblichen Zusatzbestandteile enthalten, zum Beispiel Lösungsmittel, wie Wasser und niedere aliphatische Alkohole, beispielsweise Ethanol, Propanol und Isopropanol, oder Glykole wie Glycerin und 1,2-Propylenglykol; Netzmittel oder Emulgatoren aus den Klassen der anionischen, kationischen, amphoteren oder nichtionogenen oberflächenaktiven Tenside, wie Fettalkoholsulfate, Alkylbenzolsulfonate, Alkyltrimethylammoniumsalze, Feuchthaltemittel, Alkylbetaine, oxethylierte Fettalkohole, oxethylierte Nonylphenole, Fettsäurealkanolamide, oxethylierte Fettsäureester, in einer Menge von 0,1 bis 30 Gewichtsprozent, Parfümöle in einer Menge von 0,1 bis 0,5 Gew.%; Trübungsmittel, wie zum Beispiel Ethylenglykoldistearat, in einer Menge von etwa 0,2 bis 5,0 Gew.%; Perlglanzmittel, wie zum Beispiel ein Gemisch aus Fettsäuremonoalkylolamid und Ethylenglykoldistearat, in einer Menge von etwa 1,0 bis 10 Gew.%; bakterizide und fungizide Wirkstoffe; wie zum Beispiel 2,4,4-Trichlor-2-hydroxydiphenylether oder Methylchlorisothiazolion, in einer Menge von 0,01 bis 1,0 Gew.%; Verdickungsmittel, wie beispielsweise Kokosfettsäurediethanolamid, in einer Menge von etwa 0,2 bis 3,0 Gew.%, Puffersubstanzen, wie beispielsweise Natriumcitrat oder Natriumphosphat, in einer Menge von 0,1 bis 1,0 Gew.%; Lösungsvermittler, wie zum Beispiel ethoxyliertes Rizinusöl, in einer Menge von etwa 0,1 bis 1,0 Gew.%; Anfärbestoffe, wie zum Beispiel Fluorescein-Natriumsalz, in einer Menge von etwa 0,1 bis 1,0 Gew.%; Pflegestoffe, wie zum Beispiel Pflanzen- und Kräuterextrakte, Protein- und Seidenhydrolysate, kationische Harze, Lanolinderivate, in einer Menge von 0,1 bis 5 Gew.%; physiologisch verträgliche Silikonderivate, wie zum Beispiel Silikonöl, Silikonpolymere und Siloxane; Lichtschutzmittel, Antioxidantien, Radikalfänger, Antischuppenwirkstoffe, in einer Menge von etwa 0,01 bis 2 Gew.%; physiologisch verträgliche organische Säuren, wie zum Beispiel Ameisensäure, Glyoxylsäure, Milchsäure, Weinsäure, Zitronensäure, natürliche, modifizierte natürliche oder synthetische Polymere, wie zum Beispiel Schellack, kationische, anionische, nichtionische, amphotere Polymere, Hydroxycellulose, Chitosan, Chitin oder Chitosanderivate; direktziehende Haarfarbstoffe, Haarfarbstoffe, die oxidativ entwickelt werden, Oxidationsmittel, Reduktionsmittel, Fettalkohole, Glanzgeber, Vitamine, Weichmacher, Kämmbarkeitsverbesserer, rückfettende Agenzien, Entschäumer sowie Treibgase, wie zum Beispiel Fluorkohlenwasserstoffe, Dimethylether, Kohlenwasserstoffe und komprimierbare Gase.

Wenn das aus den Instantgel-Zubereitungen herstellbare Mittel als Haar- und/oder Körperreinigungsmittel, beispielsweise als Shampoo oder Duschgel vorliegt, so enthält es mindestens ein waschaktives Tensid oder Tensidgemisch.

Geeignete anionische Tenside sind z.B. Alkylsulfate, Alkylethersulfate, alpha-Olefinsulfonate, Sulfosuccinate wie Disodium Laureth-3 Sulfosuccinat, Disodium PEG-5 Laurylcitrat Sulfosuccinat, Disodium Ricinolamido MEA-Sulfosuccinat oder Disodium Laurylamido MEA-Sulfosuccinat und Ethercarboxylate wie z.B. Sodium Laureth-6 Carboxylat oder Sodium Laureth-11 Carboxylat.

Geeignete nicht-ionische Tenside sind z.B. alkoxylierte Fettalkohole mit einem hohen Alkoxylierungsgrad, z.B. von 11 bis 50 sowie alkoxylierte Fettsäureester, alkoxylierte Partialglyceride von verzweigten oder unverzweigten, gesättigten oder ungesättigten C6- bis C20-Fettsäuren und einem Alkoxylierungsgrad von 11 bis 400 wie z.B. Polyethylenglykol(200)glycerylpalmitat, alkoxylierte Polyolester wie z.B. ethoxylierte Zuckerester, beispielsweise Polyethylenglykol(120)methyl-glucosedioleat und Alkylpolyglucoside wie z.B. Coco-Glucoside, Lauryl-Glucoside oder Decyl-Glucoside. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Auch die ethoxylierten Fettalkohole, die unter der Typenbezeichnung Dehydol® von der Firma Henkel oder unter der Typenbezeichnung Brij® von der Firma ICI Surfactants vertrieben werden, sind für das erfindungsgemäße Haarbehandlungsmittel geeignet, sofern sie einen genügend hohen Ethoxylierungsgrad aufweisen.

Unter den Fettsäureesterethoxylaten sind vor allem Diglyceridethoxylate und ethoxylierte Rizinusöle zu nennen wie z.B. PEG-25 Hydrogenated Castor Oil, PEG-35 Castor Oil, PEG-40 Hydrogenated Castor Oil.

Weiterhin können die als nicht-ionische Tenside bekannten ethoxylierten Fettsäurezuckerester, insbesondere der ethoxylierte Sorbitanfettsäureester, aber auch nicht ethoxylierte Tenside, wie die Fettsäurezuckerester, die von der Firma ICI Surfactants unter dem Handelsnamen Tween® und Arlacel® vertrieben werden sowie die Alkylpolyglycoside, die von der Firma Henkel unter dem Handelsnamen Plantaren® oder Plantacare® oder von der Firma Seppic unter dem Handelsnamen Oramix® vertrieben werden, für die erfindungsgemäße kosmetische Zubereitung eingesetzt werden.

Geeignete amphotere Tenside sind beispielsweise Betaine wie Cocamidopropylbetain oder Laurylbetain, Sulfobetaine wie z.B. Cocamidopropyl Hydroxysultaine, Glycinate wie z.B. Cocoamphoglycinat (INCI-Bezeichnung: Sodium Cocoamphoacetate) und -diglycinat sowie Propionate wie z.B. Cocoamphopropionat.

Wenn das aus den Instantgel-Zubereitungen herstellbare Mittel als Stylingmittel für Haare vorliegt, so enthält es vorzugsweise zusätzlich mindestens ein filmbildendes und haarfestigendes Polymer. Die filmbildenden Polymere liegen vorzugsweise in einer Menge von 0,01 bis 25, besonders bevorzugt von 0,1 bis 20 Gew.% vor und können einzeln oder in einem Gemisch eingesetzt werden und in gelöster Form oder als Dispersion vorliegen. Unter filmbildenden und haarfestigenden Polymeren sollen solche Polymere verstanden werden, die in der Lage sind, auf Haaren einen Polymerfilm abzuscheiden und auf diese Weise das Haar zu festigen. Die filmbildenden Polymere können nichtionisch, kationisch, anionisch, zwitterionisch oder amphoter sein und können synthetischen oder natürlichen Ursprungs sein. Ein derartiges Mittel kann bei Einsatz von festigenden Polymeren in Kombination mit gasifizierten, zuckerumhüllten Teilchen als Haarfestigungsmittel verwendet werden.

Geeignete anionische Polymere sind synthetische Homo- oder Copolymere mit neutralisierbare Säuregruppen enthaltenden Monomereinheiten, welche gegebenenfalls mit Comonomeren, die keine Säuregruppen enthalten, copolymerisiert sind. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes anionisches, natürliches Polymer ist beispielsweise teilweise oder vollständig neutralisierter Schellack.

Bevorzugte Polymere mit Säuregruppen sind vernetzte oder unvernetzte Vinylacetat/Crotonsäure Copolymere, die beispielsweise in Form einer 60%igen Lösung in Isopropanol/Wasser unter der Handelsbezeichnung ARISTOFLEX® von der Firma HOECHST/Deutschland beziehungsweise von der Firma BASF unter dem Handelsnamen LUVISET® CA-66 vertrieben werden. Weitere geeignete anionische Polymere sind zum Beispiel Terpolymere aus Vinylacetat, Crotonsäure und Polyethylenoxid sowie Terpolymere aus Acrylsäure, Alkylacrylat und N-Alkylacrylamid, insbesondere Acrylsäure/Ethylacrylat/N-t-Butylacrylamid Terpolymere, wie sie unter den Handelsnamen ULTRAHOLD® 8 und ULTRAHOLD® STRONG der Firma BASF/Deutschland vertrieben werden oder Terpolymere aus Vinylacetat, Crotonat und Vinylalkanoat, insbesondere Vinylacetat/Crotonat/Vinylneodecanoat Copolymere, wie sie z.B. von der Firma National Starch unter der Handelsbezeichnung RESYN 28-2930 vertrieben werden.

Eine weitere Klasse von geeigneten anionischen Polymeren sind anionische Polyurethane. Bevorzugte Polyurethane sind dadurch gekennzeichnet, dass sie (a) endständige Säuregruppen besitzen, die beispielweise über Aminosulfonsäuren oder Aminocarbonsäuren eingeführt wurden, (b) gegebenenfalls weitere freie Carbonsäuregruppen enthalten, die durch Einpolymerisieren von Carbonsäurediolen wie z.B. Dimethylolpropansäure als Comonomere eingeführt wurden und (c) Polyurethansequenzen enthalten, die aus Polyesterdiolen und Diisocyanaten wie beispielsweise Alkylendiisocyanaten oder Isophorondiisocyanat gebildet wurden. Geeignet ist beispielsweise Luviset® PUR der Firma BASF/Deutschland.

Die anionischen Polymere liegen in dem aus den Instantgel-Zubereitungen herstellbaren Mittels teilweise oder vollständig mit einem kosmetisch verträglichen Neutralisationsmittel neutralisiert vor. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH u.a..

Geeignete synthetische, nichtionische, filmbildende, haarfestigende Polymere sind zum Beispiel Homo- oder Copolymere, welche aus mindestens einem nichtionischen Monomer aufgebaut sind. Nichtionische Monomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, wobei die Alkylgruppen dieser Monomere vorzugsweise C1- bis C7-Alkylgruppen, besonders bevorzugt C1-bis C3-Alkylgruppen sind. Geeignete synthetische, nichtionische, filmbildende, haarfestigende Polymere sind zum Beispiel Homopolymere des Vinylpyrrolidons sowie Homopolymere des N-Vinylformamids. Weitere geeignete synthetische filmbildende, nichtionische haarfestigende Polymere sind zum Beispiel Copolymerisate aus Vinylpyrrolidon und Vinylacetat, Terpolymere aus Vinylpyrrolidon, Vinylacetat und Vinylpropionat, Polyacrylamide, Polyvinylalkohole oder Polyethylenglykole mit einem Molekulargewicht von 800 bis 20.000 g/mol.

Geeignete natürliche filmbildende Polymere sind zum Beispiel Chitosan mit einem Molekulargewicht von 20.000 bis ca. 5 Millionen g/mol, wie es beispielsweise von der Firma Pronova vertrieben wird, oder verschiedene Saccharidtypen wie zum Beispiel Polysaccharide oder Gemische aus Oligo-, Mono- und Disacchariden, welche beispielsweise unter dem Handelsnamen C-PUR® von der Firma Cerestar, Brüssel/Belgien vertrieben werden. Weitere geeignete, natürliche Polymere sind chinesiches Balsamharz und Cellulosederivate, zum Beispiel Hydroxypropylcellulose mit einem Molekulargewicht von 30.000 bis 50.000 g/mol.

Geeignete filmbildende kationische Polymere sind dadurch gekennzeichnet, dass sie aus mindestens einer Monomerart aufgebaut sind, die kationische oder kationisierbare Gruppen, vorzugsweise primäre, sekundäre, tertiäre oder quaternäre Stickstoffgruppen enthält. Geeignete ammoniumsubstituierte Vinylmonomere sind zum Beispiel Trialkylmethacryloxyalkylammonium, Trialkylacryloxyalkylammonium, Dialkyldiallylammonium, quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, welches am heterocyclischen Ring mit bis zu 3 C1- bis C12-Alkylresten substituiert sein kann, Alkylvinylpyridinium, oder Alkylvinylpyrrolidon Salze. Geeignete aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, N-Vinylimidazol, welches am Ring mit bis zu 3 C1- bis C12-Alkylresten substituiert sein kann. Die Alkylgruppen dieser Monomere sind vorzugsweise niedere Alkylgruppen wie zum Beispiel C1- bis C7-Alkylgruppen, besonders bevorzugt C1- bis C3-Alkylgruppen. Die kationischen bzw. basischen Monomere können mit nicht-kationischen bzw. nicht-basischen Comonomeren copolymerisiert sein.

Geeignete kationische Polymere sind zum Beispiel Polyvinylpyrrolidon/Dimethylaminoethylmethacrylat Copolymer, ein Copolymer aus Polyvinylpyrolidon und Imidazoliminmethochlorid, ein Terpolymer aus Dimethyldiallylammoniumchlorid, Natriumacrylat und Acrylamid, ein Terpolymer aus Vinylpyrrolidon, Dimethylaminoethylmethacrylat und Vinylcaprolactam, mit quaternierten Ammoniumgruppen substituierte Hydroxyethylcellulose, Vinylpyrrolidon/Methacrylamidopropyltrimethylammoniumchlorid Copolymer oder diquaternäre Polydimethylsiloxane (INCI: Quaternium-80).

Geeignete amphotere Polymere sind Polymere, welche sowohl kationische oder durch Protonierung kationisierbare Gruppen als auch anionische oder durch Deprotonierung anionisierbare Gruppen enthalten. Kationische Gruppen sind beispielsweise quaternäre Amingruppen, kationisierbare Gruppen sind beispielsweise primäre, sekundäre oder tertiäre Amingruppen. Anionische Gruppen sind beispielsweise Carboxylat-, Sulfat-, Sulfonat-, Phosphat- oder Phosphonatgruppen. Anionisierbare Gruppen sind beispielsweise die protonierten Formen der genannten anionischen Gruppen.

Geeignete amphotere Polymere sind z.B. Copolymere aus Octylacrylamid, t-Butylaminoethylmethacrylat und zwei oder mehr Monomeren, bestehend aus Acrylsäure, Methacrylsäure oder deren Estern. Weitere Beispiele sind Copolymere von Acrylsäure, Methylacrylat und Methacrylamidopropyltrimethylammoniumchlorid (INCI: Polyquaternium-47), Copolymere aus Acrylamidopropyltrimoniumchlorid und Acrylaten oder Copolymere aus Acrylamid, Acrylamidopropyltrimoniumchlorid, 2-Amidopropylacrylamidsulfonat und DMAPA (INCI: Polyquaternium-43).

Wenn das aus den Instantgel-Zubereitungen herstellbare Haarbehandlungsmittel in Form eines Haargels vorliegt, so enthält es zusätzlich mindestens eine gelbildende Substanz, beispielsweise eines Verdickungsmittels ausgewählt aus vernetzten oder unvernetzten Homopolymeren der Acrylsäure, insbesondere Carbopolen, Acrylsäure/Acrylamid Copolymeren und natürlichen Verdickern wie, Xanthan Gum, Cellulosederivaten etc. Die Verdicker sind in einer Menge von vorzugsweise 0,05 bis 10, besonders bevorzugt von 0,1 bis 2 Gew.% enthalten. Die Viskosität des Gels beträgt vorzugsweise von 500 bis 50.000 cSt, besonders bevorzugt von 1.000 bis 15.000 cSt bei 25°C (gemessen mit einem Rotationsviskosimeter nach DIN 53 018 T1 u. 2).

Wenn das aus den Instantgel-Zubereitungen herstellbare Haarbehandlungsmittel in Form einer Haarlotion vorliegt, so liegt es als im wesentlichen nicht-viskose oder gering viskose, fließfähige Lösung, Dispersion oder Emulsion mit einem Gehalt an mindestens 10 Gewichtsprozent, vorzugsweise 20 bis 95 Gewichtsprozent eines kosmetisch verträglichen Alkohols vor. Als Alkohole können insbesondere die für kosmetische Zwecke üblicherweise verwendeten niederen Alkohole mit 1 bis 4 Kohlenstoffatomen wie zum Beispiel Ethanol und Isopropanol verwendet werden.

Wird das aus den Instantgel-Zubereitungen herstellbare Mittel in Form eines Haartönungsmittels eingesetzt, so enthält es zusätzlich 0,05 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 1,4-Diamino-2-nitrobenzol, Azofarbstoffe, zum Beispiel Acid Brown 4 (C. I. 14 805), Anthrachinonfarbstoffe, zum Beispiel Disperse Voilet 4 (C. I. 61 105), Triphenylmethanfarbstoffe, zum Beispiel Basic Violet 1 (C. I. 42 535), wobei die Farbstoffe je nach Art ihrer Substituenten sauren, nichtionischen oder basischen Charakter haben können und/oder natürliche Haarfarbstoffe, wie zum Beispiel Henna oder Reng, der zur Farbentwicklung nicht der Oxidation bedarf.

Die Anwendung des aus den Instantgel-Zubereitungen herstellbaren Mittels zur Behandlung von Haaren erfolgt entweder, indem in einem ersten Schritt ein wasserhaltiges Haarbehandlungsmittel vermischt wird mit Teilchen, welche mindestens ein in einer festen, wasserlöslichen Umhüllung auf Saccharidbasis eingeschlossenes Gas enthalten und die Mischung in einem zweiten, unmittelbar anschließenden Schritt auf das Haar aufgetragen wird. Die Anwendung kann aber auch erfolgen, indem in einem ersten Schritt das Haar gewaschen, gespült oder anderweitig mit Wasser angefeuchtet wird und in einem zweiten Schritt ein wasserfreies Mittel, z.B. ein Trockenshampoo auf das feuchte Haar aufgetragen wird, wobei das wasserfreie Mittel aus gasifizierten Teilchen besteht oder gasifizierte Teilchen enthält, welche ein in einer festen, wasserlöslichen Umhüllung auf Saccharidbasis eingeschlossenes Gas enthalten. Vorzugsweise werden in feuchtem oder nassem Haar je nach Haarfülle 5 bis 30 g des Mittels verteilt, wobei vorzugsweise 0,5 bis 6 g bzw. 10 bis 20 %, besonders bevorzugt 1 bis 3 g der Gesamtmenge die gasifizierten Teilchen sind. Anschließend wird das Haar durchgekämmt und zur Frisur geformt und getrocknet.

Als Gelbildner (A) geeignet sind prinzipiell alle in bekannten Instantprodukten üblicherweise eingesetzten, viskositätserhöhend oder gelierend wirkenden, ohne Verklumpen leicht wasserlöslichen oder leicht wasserquellbaren Stoffe wie z.B. vorgelierte Stärke und vorgelierte Stärkederivate.

Bevorzugte Gelbildner sind superabsorbierende Polymere. Hierzu zählen Polyacrylate oder Polymethacrylate, insbesondere deren Natriumsalze, welche hochmolekular (MW z.B. > 1.000.000) und vernetzt sind. Bei Kontakt mit Wasser nehmen sie ein Vielfaches ihres Eigengewichtes an Wasser auf und es bilden sich gelierte Partikel. Die superabsorbierenden Polymere bilden bei Kontakt mit Wasser bzw. wässrigen Lösungen gequollene, gelierte Partikel, welche in dem gebrauchsfertigen Gel in dispergierter oder assoziierter Form vorliegen. Superabsorbierende Polymere sind bekannt durch ihre Verwendung als Absorber für Flüssigkeiten, beispielsweise in absorbierenden Sanitärartikeln wie Babywindeln, bei der Erwachseneninkontinenz, der Damenhygiene und der Wundabdeckung. Sie können definiert werden als wasserunlösliche, vernetzte Polymere, die in der Lage sind, unter Quellung und Ausbildung von Hydrogelen ein mehrfaches, d.h. bis zum 1000-fachen ihres Eigengewichts an wässrigen Flüssigkeiten aufzunehmen und die absorbierte Flüssigkeitsmenge unter Druck zurückzuhalten. Hierbei handelt es sich üblicherweise um Polymere oder hydrophile Copolymere von Acryl- oder Methacrylsäure oder um Pfropfcopolymere aus Stärke und Acrylsäure, wobei die Polymere neutralisiert oder teilneutralisiert als Salze vorliegen können und typischerweise hochmolekular (z.B. MW > 1.000.000) sind. Sie werden gebildet durch Polymerisation unter teilweiser Vernetzung mit geeigneten Vernetzern aus ethylenisch ungesättigten hydrophilen Monomeren, insbesondere Acrylsäure, Methacrylsäure oder deren Alkalisalzen. Derartige Polymere sind an sich bekannt und deren Herstellung ist vielfach beschrieben, exemplarisch wird auf die EP 0 312 952, die DE 44 18 818 und auf die EP 0 441 507 verwiesen. Die superabsorbierenden Polymere zeichnen sich durch ihr großes Wasseraufnahmevermögen und ihr großes Wasserrückhaltevermögen aus. Sie sind im Handel in Pulver- oder Granulatform erhältlich. Geeignete superabsorbierende Polymere sind beispielsweise AQUA-KEEP® D (Elf Atochem S.A.), Sanwet® IM 7015 (BASF AG) oder Sanwet® 3746-5 (BASF AG). Die mittlere Teilchengröße der trockenen Polymere beträgt vorzugsweise 100 bis 850 *µ*m. Besonders bevorzugt werden allerdings kleinere Teilchengrößen von 200 *µ*m oder darunter. Das Aufnahmevermögen für entsalztes Wasser (Centrifuge Retention Capacity) liegt vorzugsweise bei mindestens 20 g/g.

Die Gelbildner (A) sind vorzugsweise in einer Menge von 0,05 bis 20 Gew.%, besonders bevorzugt von 0,1 bis 2 Gew.%, ganz besonders bevorzugt von 0,3 bis 1 Gew.% in dem gebrauchsfertigen Gel enthalten und stellen vorzugsweise den einzigen Gelbildner des gebrauchsfertigen Gels dar. Die den akustisch wahrnehmbaren Effekt produzierenden Stoffe (B) sind vorzugsweise in einer Menge von 0,1 bis 20 Gew.%, besonders bevorzugt von 0,5 bis 10 Gew.%, ganz besonders bevorzugt von 1 bis 2 Gew.% in dem gebrauchsfertigen Gel enthalten.

Das Mengenverhältnis von Gelbildner (A) und den akustischen Effekt produzierenden Stoff (B) wird in Abhängigkeit von der Gelbildungsgeschwindigkeit des Gelbildners so gewählt, dass die Erzeugung des akustischen Effekts nach Zugabe von Wasser oder eines wasserhaltigen Lösungsmittels zu dem Zeitpunkt beendet ist, zu dem die Zubereitung eine gebrauchsfertige Konsistenz erreicht hat, d.h. im wesentlichen vollständig ausgeliert ist und sich die Viskosität nicht mehr oder nur noch unwesentlich ändert. Dieser Zeitpunkt ist vorteilhafterweise in 1 bis 60 Minuten, vorzugsweise in 1 bis 15 Minuten nach Kontakt der trockenen Instantgel-Zubereitung mit Wasser bzw. einem wasserhaltigen Lösungsmittel erreicht. Geeignete Mengenverhältnisse bewegen sich im Bereich von (A):(B) = 1:0,5 bis 1:20, vorzugsweise von 1:1 bis 1:10.

Anwendbar ist die erfindungsgemäße Instantgel-Zubereitung beispielsweise zur Herstellung von kosmetischen, pharmazeutischen oder medizinischen Mitteln oder von Nahrungsmitteln. Ein weiterer Gegenstand der Erfindung ist daher ein Verfahren zur Herstellung eines kosmetischen Mittels mit Akustikindikator, welches die erfindungsgemäße Instant Zubereitung enthält oder aus ihr besteht. Das aus den Instantgel-Zubereitungen herstellbare kosmetische Mittel enthält vorteilhafterweise weitere Zusatz- und Wirkstoffe ebenfalls in fester Form als Pulver oder Granulat, welche sich unter Rühren in Wasser leicht und schnell auflösen oder gleichmäßig dispergieren lassen. Infrage kommen hierfür z.B. feste Detergenzien oder Detergenzgemische, Farbstoffe, mikroverkapselte Parfüm-Aroma- oder Pflegestoffe etc.. Diese zusätzlichen Hilfs- und Wirkstoffe können in Mengen von 0,01 bis 5 Gew.% eingesetzt werden.

Zusätzliche Wirk- und Hilfsstoffe, welche in flüssiger Form vorliegen wie z.B. Parfümöle, werden vorteilhafterweise in mikroverkapselter Form eingesetzt. Die Mikroverkapselung von Stoffen, insbesondere auch von Parfümölen, ist an sich bekannt, vgl. z.B. die Literatur Seifen-Öle-Fette-Wachse, 115. Jg. Nr. 3 (1989), S.93-98. Das Kapselmaterial kann dabei so beschaffen sein, dass es gegenüber einem wässrigen Medium stabil ist und die Kapselinhaltsstoffe bei der Anwendung durch mechanische Einwirkung, z.B. durch Verreiben mit den Händen, freigesetzt werden. Das Kapselmaterial kann aber auch so beschaffen sein, dass es sich innerhalb von 1 bis 60 Minuten, vorzugsweise 1 bis 15 Minuten teilweise oder vollständig auflöst und dadurch die Inhaltsstoffe in dem gebrauchsfertigen Gel freisetzt. Die den akustisch wahrnehmbaren Effekt produzierenden Stoffe (B) können daher auch als akustischer Indikator für die Gebrauchsfertigkeit hinsichtlich der ausreichenden Freisetzung von Hilfs- und Wirkstoffen einer zunächst wasserfreien, Wirk- und Hilfsstoffe enthaltenden Zubereitung nach Zugabe von Wasser dienen, wobei die Wirk- und Hilfsstoffe in Mikrokapseln aus in wasserhaltigen Medien instabilem Kapselmaterial vorliegen. Ein geeignetes, lösliches Kapselmaterial ist beispielsweise Cyclodextrin. Der Durchmesser der Mikrokapseln beträgt vorteilhafterweise 5 bis 2000 *µ*m, bevorzugt 75 bis 250 *µ*m.

Geeignete pulverförmige Tenside, z.B. zur Herstellung eines aus den Instantgel-Zubereitungen herstellbaren Reinigungsmittels, sind in der DE 197 45 964 genannt. Geeignete anionische Detergenzien sind sulfonierte oder sulfatierte Alkyl-, Aralkyl- oder Alkylaryldetergenzien, Alkylethersulfate, Alkylethersulfonate, Alkylsuccinate, Alkylsulfosuccinate, Alkylestersulfonate, N-Alkoylsarcosinate, Methyltaurate, Taurate und Isethionate, sofern sie in fester Form vorliegen und eine ausreichende Wasserlöslichkeit aufweisen, insbesondere Natrium-, Magnesium-, Ammonium- und Mono-, Di- oder Triethanolaminsalze von Alkyl- oder Arylsulfaten sowie entsprechende Salze von Alkarylsulfonaten, wobei die Alkylgruppen im allgemeinen 12 bis 21 C-Atome aufweisen und ungesättigt oder vorzugsweise gesättigt sein können. Als kationische Detergenzien kommen monoquaternäre oder bisquaternäre Ammoniumverbindungen in Frage, die mindestens einen langkettigen aliphatischen Rest mit 10 bis 26 C-Atomen tragen. Als nicht-ionische Detergenzien können Kondensationsprodukte aus Ethylenoxid oder Propylenoxid mit einem langkettigen Alkohol, einem langkettigen Amin oder einer langkettigen Carbonsäure eingesetzt werden, sofern sie nicht flüssig und ausreichend wasserlöslich sind. Dabei umfaßt die Kohlenstoffkette in der Regel 8 bis 20 C-Atome und kann mit mindestens 2, vorzugsweise mit 5 bis 20 Ethylenoxid- oder Propylenoxideinheiten kondensiert sein. Als nichtionische Detergenzien können auch Alkylpolyglykoside mit 8 bis 14 C-Atomen in der Alkylkette eingesetzt werden, z.B. Laurylpolyglucose. Als amphotere oder zwitterionische Detergenzien werden vor allem Betaine eingesetzt, die mindestens eine langkettige Alkylgruppe tragen. Hierzu gehören Alkylamidopropylbetaine, Alkylbetaine, Alkylamphoacetate und -diacetate, Sulfobetaine und Phosphobetaine, wobei die Alkylgruppen 8 bis 20 C-Atome aufweisen, sofern sie in fester Form vorliegen und eine ausreichende Wasserlöslichkeit aufweisen. Insbesondere geeignet sind Cocosdimethylcarboxymethylbetain, Lauryldimethylcarboxymethylbetain, Lauryldimethyl-α-carboxyethylbetain, Cetyldimethyl-carboxymethylbetain, aber auch Sulfobetaine wie Cocosdimethylsulfodimethylbetain, Amidoalkylbetaine und Amidoalkylsulfobetaine, wobei die Carboxybetaine und Amidobetaine bevorzugt sind. Spezielle Beispiele hierfür sind Cocosamidopropylbetain, Lauramidopropylbetain, Myristylamidopropylbetain und deren Mischungen.

Geeignete feste Farbstoffe sind beispielsweise solche, wie sie üblicherweise zum Anfärben von Lebensmitteln oder von Kosmetika eingesetzt werden, z.B. Acid Blue 9 (Food Blue 2, CI 42090), Acid Red 51 (Food Red 14, CI 45430), Acid Yellow 3 (Food Yellow 13, CI 47005) oder Acid Green 25 (CI 61570).

Dadurch, dass die Zuckerumhüllung der gasifizierten Teilchen selbst eine hohe Affinität zum Haar hat, kann nach einer Behandlung von Haaren mit dem erfindungsgemäßen kosmetischen Mittel ein positiver Volumen- oder Stylingeffekt des behandelten Haars festgestellt werden. Zur Steigerung eines haarfestigenden Effektes können aber noch weitere feste, schnell wasserlösliche, haarfestigende Stoffe zugefügt werden, beispielsweise weitere Saccharide oder Polysaccharide wie z.B. Glucose.

Die Viskosität des gebrauchsfertigen Gels beträgt vorzugsweise von 500 bis 3000 mPa s, besonders bevorzugt von 1000 bis 1500 mPa s (gemessen mit einem Rotationsviskosimeter RheoStress 100 der Firma Haake bei einer Temperatur von 25°C und einem Schergefälle von 0,5 bis 1400 s⁻¹).

Die Anwendung der erfindungsgemäßen Instantgel-Zubereitung bzw. des aus den Instantgel-Zubereitungen herstellbaren kosmetischen Mittels erfolgt, indem entweder Wasser oder ein wasserhaltiges Lösungsmittelsystem vorgelegt und die erfindungsgemäße Instantgel-Zubereitung zugegeben wird oder wobei die erfindungsgemäße Instantgel-Zubereitung bzw. das diese Zubereitung enthaltende kosmetische Mittel vorgelegt und mit Wasser oder einem wasserhaltigen Lösungsmittel versetzt wird. Anschließend wird solange gerührt, bis die akustischen Effekte beendet und/oder das Mittel seine gebrauchsfertige Konsistenz erreicht hat. Danach kann das verdickte oder gelierte Produkt seiner bestimmungsgemäßen Anwendung zugeführt werden.

In einer besonders bevorzugten Ausführungsform enthält die erfindungsgemäße Instantgel-Zubereitung in fester Form mindestens ein superabsorbierendes Polymer sowie gasifizierte Teilchen, welche aus einer festen Umhüllung auf Saccharidbasis und darin eingeschlossenem Kohlendioxid bestehen. Weitere Zusatz-, Hilfs- oder Wirkstoffe können in fester, pulverförmiger, granulatförmiger oder mikroverkapselter Form enthalten sein.

Die nachfolgenden Beispiele sollen den Gegenstand der Erfindung näher erläutern.

### Beispiele

In den folgenden Beispielen wurden als gasifizierte Teilchen das Produkt 'Popping Candy Nature Dust' der Firma Zeta Espacial S.A., Rubi/Spanien verwendet. Hierbei handelt es sich um Teilchen, welche 0,3 bis 2,0 ml Kohlendioxid pro Gramm in einer Umhüllung aus Zucker, Lactose und Glucose enthalten und eine Größe von ca. 0,5 bis 4,5 mm aufweisen.

### Beispiel 1: Refreshing Gel

### Instantgelzubereitung:

| | |
|---|---|
| 0,5 g | Sanwet® 3746-5 (BASF, Superabsorbierendes Natriumpolyacrylat) |
| 1,5 g | Popping Candy |
| 0,1 g | Mikroverkapseltes Parfüm |

Die Instantgelzubereitung wird unter Rühren zu einer solchen Menge Wasser zugegeben, dass die Gesamtmenge 100 g beträgt. Es bildet sich nach 2-3 Minuten Quellzeit ein gebrauchsfertiges Gel.

### Beispiel 2: Gel mit haarfestigender Wirkung

### Instantgelzubereitung:

| | |
|---|---|
| 0,7 g | Sanwet® 3746-5 (BASF, Superabsorbierendes Natriumpolyacrylat) |
| 2,0 g | Popping Candy |
| 0,1 g | Mikroverkapseltes Parfüm |
| 2,5 g | C-PUR® 1934 (Glucose) |

Die Instantgelzubereitung wird unter Rühren zu einer solchen Menge Wasser zugegeben, dass die Gesamtmenge 100 g beträgt. Es bildet sich nach 2-3 Minuten Quellzeit ein gebrauchsfertiges Gel.

### Beispiel 3: 2-Phasen Haarglanzgel

### Instantgelzubereitung (Phase 1):

| | |
|---|---|
| 0,5 g | Sanwet® 3746-5 (BASF, Superabsorbierendes Natriumpolyacrylat) |
| 1,0 g | Popping Candy |
| 0,1 g | Mikroverkapseltes Parfüm |

### Phase 2:

| | |
|---|---|
| 5,0 g | Glycerin |
| 93,4 g | Wasser |

Die Instantgelzubereitung (Phase 1) wird zu Phase 2 zugegeben und verrührt. Es bildet sich nach 2-3 Minuten Quellzeit ein gebrauchsfertiges Gel.

## Patentansprüche

1. Instantgel-Zubereitung bestehend aus einem Gemisch von Trockensubstanzen, wobei das Gemisch einen Gehalt aufweist an
(A) mindestens einem Gelbildner und/oder mindestens einem superabsorbierenden Polymer und
(B) mindestens einem festen, bei Kontakt mit Feuchtigkeit einen akustisch wahrnehmbaren Effekt produzierenden Stoff, ausgewählt aus gasifizierten Teilchen, welche mindestens ein in einer festen Umhüllung eingeschlossenes Gas enthalten, wobei die Umhüllung so gewählt ist, dass das Gas bei Kontakt der Umhüllung mit Wasser oder Feuchtigkeit freigesetzt wird und das Material der Umhüllung ausgewählt ist aus Materialien auf Saccharidbasis, und wobei das in den gasifizierten Teilchen eingeschlossene Gas ausgewählt ist aus Kohlendioxid, Stickstoff, Luft oder aus Mischungen dieser Gase und wobei der Druck des in den Teilchen eingeschlossenen Gases größer ist als der Umgebungsdruck.

2. Zubereitung nach Anspruch 1, **dadurch gekennzeichnet, dass** Komponente (A) ausgewählt ist aus superabsorbierenden Polymeren und vorgelierten Stärken und deren Derivaten.

3. Zubereitung nach einem der Ansprüche 1 bis 2,
**dadurch gekennzeichnet, dass** das Verhältnis von Gelbildner (A) und dem einen akustischen Effekt produzierenden Stoff (B) 1:0,5 bis 1:20 beträgt.

4. Instantgel-Zubereitung nach einem der Ansprüche 1 bis 3 enthaltend mindestens einen weiteren Zusatz-, Hilfs- oder Wirkstoff in fester, pulverförmiger, granulatförmiger oder mikroverkapselter Form.

5. Verwendung von gasifizierten Teilchen, welche mindestens ein in einer festen Umhüllung auf Saccharidbasis eingeschlossenes Gas enthalten, wobei die Umhüllung so gewählt ist, dass das Gas bei Kontakt der Umhüllung mit Wasser oder Feuchtigkeit freigesetzt wird, als akustischer Indikator zur Anzeige der Gebrauchsfertigkeit einer Instantzubereitung nach Flüssigkeitszugabe oder zur Anzeige der Gebrauchsfertigkeit einer Wirk- und Hilfsstoffe enthaltenden Zubereitung nach Zugabe von Wasser oder eines wasserhaltigen Lösungsmittels, wobei die Wirk- und Hilfsstoffe in Mikrokapseln aus nicht wasserbeständigem Material vorliegen.

6. Verwendung nach Anspruch 5, **dadurch gekennzeichnet, dass** das Gas ausgewählt ist aus Kohlendioxid, Stickstoff und Sauerstoff oder aus Mischungen dieser Gase und dass der Druck des in den Teilchen eingeschlossenen Gases größer ist als der Umgebungsdruck.

7. Verwendung nach einem der vorstehenden Verwendungsansprüche, **dadurch gekennzeichnet, dass** die Umhüllung beschichtet ist.

8. Verwendung nach Anspruch 7, **dadurch gekennzeichnet, dass** das Beschichtungsmaterial ausgewählt ist aus Fetten, Schellack, Gelatine, Cellulose oder Cellulosederivaten.

9. Verwendung nach einem der vorstehenden Verwendungsansprüche, **dadurch gekennzeichnet, dass** die Menge des eingeschlossenen Gases von 0,05 bis 15 cm³/g beträgt.

10. Verfahren zur Herstellung eines verdickten, gebrauchsfertigen kosmetischen Mittels, wobei entweder Wasser oder ein wasserhaltiges Lösungsmittelsystem vorgelegt und eine Zubereitung gemäß einem der Ansprüche 1 bis 4 zugegeben wird oder wobei zu einer Zubereitung gemäß einem der Ansprüche 1 bis 4 Wasser oder ein wasserhaltiges Lösungsmittelsystem zugegeben wird und wobei gerührt wird, bis die akustischen Effekte beendet sind.

## Claims

1. An instant gel preparation comprising a mixture of dry substances, wherein said mixture contains
(A) at least one gel former and/or at least one super-absorbent polymer and
(B) at least one solid material that produces a detectable auditory effect upon contact with moisture, selected from gasified particles containing at least one gas enclosed in a solid casing, wherein the casing is selected such that the gas is released on contact of the casing with water or moisture and the material of the casing is selected from materials based on saccharide, and wherein the gas enclosed in the gasified particles is selected from carbon dioxide, nitrogen, air, or a mixture of these gases, and wherein the pressure of the gas enclosed in said particles is greater than the ambient pressure.

2. The preparation according to Claim 1, **characterized in that** component (A) is selected from super-absorbent polymers and pre-gelled starches and their derivatives.

3. The preparation according to one of Claims 1 to 2, **characterized in that** the ratio of gel builder (A) and the material producing an auditory effect (B) is 1:0.5 to 1:20.

4. The instant gel preparation according to any one of Claims 1 through 3, containing at least one additional additive, auxiliary or active substance in solid, powder, granular, or microencapsulated form.

5. Use of gasified particles, which contain at least one gas enclosed in a solid casing based on saccharide, wherein the casing is selected such that when the casing is contacted by water or moisture the gas is released as an auditory indicator to signal that an instant preparation is ready to use following the addition of a liquid or to signal that a preparation containing active substances or auxiliaries is ready to use following the addition of water or an aqueous solvent, wherein the active substances or auxiliaries are present in microcapsules consisting of materials that are not water resistant.

6. The use according to Claim 5, **characterized in that** the gas is selected from carbon dioxide, nitrogen or oxygen, or from a mixture of these gases, and that the pressure of the gas enclosed in the particles is greater than the ambient pressure.

7. The use according to any one of the previous use claims, **characterized** and that the casing is coated.

8. The use according to Claim 7, **characterized in that** the coating material is selected from fats, shellac, gelatin, cellulose, or cellulose derivatives.

9. The use according to any one of the previous use claims, **characterized in that** the quantity of enclosed gases is from 0.05 to 15 cm³/g.

10. A method for producing a thickened, ready-to-use cosmetic agent, wherein either water or an aqueous solvent system is provided and a preparation according to any one of Claims 1 through 4 is added or wherein water or an aqueous solvent system is added to a preparation according to any of claims 1 through 4 and wherein [the mixture is] stirred until the acoustic effects cease.

## Revendications

1. Préparation de gel instantané comprenant un mélange de substances sèches, ledit mélange contenant
(A) au moins un formateur de gel et/ou au moins un polymère superabsorbant et
(B) au moins un matériau solide qui produit un effet acoustique détectable en contact avec de l'humidité, choisi parmi des particules gazéifiées contenant au moins un gaz enclavé dans une enveloppe solide, où ladite enveloppe est choisie de telle sorte que ledit gaz est libéré au contact de ladite enveloppe avec de l'eau ou de l'humidité et ledit matériau de ladite enveloppe est choisi parmi les matériaux à base de saccharide, et dans laquelle ledit gaz enclavé dans lesdites particules gazéifiées est choisi parmi le dioxyde de carbone, l'azote, l'air, ou un mélange de ces gaz, et dans laquelle la pression dudit gaz enclavé dans lesdites particules est supérieure à la pressure ambiante.

2. Préparation selon la revendication 1, **caractérisée en ce que** le composant (A) est choisi parmi les polymères superabsorbants et les amidons prégélifiés et leurs dérivés.

3. Préparation selon l'une des revendications 1 à 2, **caractérisée en ce que** le rapport d'adjuvant de gel (A) et le matériau produisant un effet acoustique (B) va de 1:0,5 à 1:20.

4. Préparation de gel instantané selon l'une quelconque des revendications 1 à 3, contenant au moins un additif supplémentaire, substance auxiliaire ou active sous forme solide, poudreuse, granulaire, ou microencapsulée.

5. Utilisation de particules gazéifiées, qui contiennent au moins un gaz enclavé dans une enveloppe solide à base de saccharide, dans laquelle ladite enveloppe est choisie de telle sorte que ledit gaz est libéré au contact de ladite enveloppe avec de l'eau ou de l'humidité en tant qu'indicateur acoustique pour signaler qu'une préparation instantanée est prête pour l'utilisation après l'addition d'un liquide ou pour signaler qu'une préparation contenant des substances actives ou des auxiliaires est prête pour l'utilisation après l'addition d'eau ou d'un solvant aqueux, dans laquelle lesdites substances actives ou auxiliaires sont présentes dans des microgélules constituées de matériaux qui ne sont pas résistants à l'eau.

6. Utilisation selon la revendication 5, **caractérisée en ce que** le gaz est choisi parmi le dioxyde de carbone, l'azote ou l'oxygène, ou un mélange de ces gaz, et **en ce que** la pression dudit gaz enclavé dans les particules est supérieure à la pression ambiante.

7. Utilisation selon l'une quelconque des revendications d'utilisation précédentes, **caractérisée en ce que** l'enveloppe est revêtue.

8. Utilisation selon la revendication 7, **caractérisée en ce que** le matériau de revêtement est choisi parmi des graisses, de la gomme laque, de la gélatine, de la cellulose, ou des dérivés de cellulose.

9. Utilisation selon l'une quelconque des revendications d'utilisation précédentes, **caractérisée en ce que** la quantité de gaz enclavés va de 0,05 à 15 cm³/g.

10. Procédé pour fabriquer un agent cosmétique épaissi, prêt à l'emploi, en fournissant ou de l'eau ou un système solvant aqueux et en ajoutant une préparation selon l'une quelconque des revendications 1 à 4 ou en ajoutant de l'eau ou un système solvant aqueux à une préparation selon l'une quelconque des revendications 1 à 4 et en agitant [le mélange] jusqu'à ce que les effets acoustiques soient terminés.
